Europäisches Patentamt

European Patent Office

Office européen des brevets

(11) Publication number: **0 264 748**
**A2**

# EUROPEAN PATENT APPLICATION

(21) Application number: **87114804.5**

(51) Int. Cl.⁴: **C12N 5/00**

(22) Date of filing: **10.10.87**

(30) Priority: **10.10.86 US 917984**

(43) Date of publication of application:
**27.04.88 Bulletin 88/17**

(84) Designated Contracting States:
**AT BE CH DE ES FR GB GR IT LI LU NL SE**

(71) Applicant: **INSTITUTE OF MOLECULAR**
**BIOLOGY Inc.**
**812 Huntington Avenue**
**Boston MA 02115(US)**

Applicant: **Rafajko, Robert R.**
**1007 Samplers Way**
**Rockville, MD 20854(US)**

(72) Inventor: **Antoniades, Harry N.**
**21 Magnolia Avenue**
**Newton, MA 02158(US)**
Inventor: **Rafajko, Robert R.**
**1007 Samplers Way**
**Rockville, MD 20854(US)**

(74) Representative: **Heidrich, Udo, Dr. jur.,**
**Dipl.-Phys.**
**Franziskanerstrasse 30**
**D-8000 München 80(DE)**

(54) Cell culture medium made from cohn supernatant fraction v.

(57) A cell culture medium that includes Cohn Supernatant Fraction V. Preferably, the medium is virus-free.

EP 0 264 748 A2

## Background of the Invention

This invention relates to the culturing of mammalian cells.

Mammalian cells are grown in culture for a variety of purposes. One increasingly important use of cultured mammalian cells is in the production of therapeutic proteins, e.g., tissue plasminogen activator, coded for by recombinant DNA - containing plasmids Examples of currently important production cell lines are mouse C127 cells and Chinese Hamster Ovary cells.

Another instance in which mammaliam cells are cultured for therapeutic purposes is the cancer treatment method described in Rosenberg et al. (1985) New Eng. J. Med. 313, 1485. According to this method, human lymphocytes are separated out of blood, cultured, treated with interleukin-2 to induce lymphocyte proliferation and activation, and infused into the patient so that the tumor is attacked by the activated lymphocytes.

Yet another important application of mammalian cell culture is the growing of hybridomas to produce therapeutically and diagnostically useful monoclonal antibodies.

In all mammalian cell culture procedures in which the cell products or the cells themselves are to be administered to a human patient, it is important that the medium be free from viruses and mycoplasma, both of which can pass through conventional sterilizing filters; hepatitis and AIDS viruses are of particular concern. This is, however, difficult to achieve, because most mammalian cells required animal serum to grow and the necessary proteins are damaged by the heating required to kill viruses and mycoplasmas.

## Summary of the Invention

We have discovered that a superior, economical mammalian cell culture medium can be provided by using, rather than serum, a human plasma fraction, known as Cohn Supernatant Fraction V, which is inexpensive, stable under virus-killing heating conditions (60°C for 20 hours), and is very effective in promoting growth of non-adherent lymphoid and hybridoma cells and maintenance of adherent cells such as human fibroblasts.

Supernatant Fraction V is a fraction produced during Method 6 of the Cohn Plasma Fractionation Process (described in Cohn et al. J. Am. Chem. Soc: 68, 459 (1946), and later in Hormones in Human Plasma, Harry N. Antoniades, ed., Little Brown and Company, Boston, 19_ ). The Cohn process is carried out by licensed laboratories according to the Bureau of Biological Standards's strictly defined procedures which, from time to time, with government approval, are varied slightly. Supernatant Fraction V, the fraction which we have discovered is valuable in mammalian cell culture, is commonly discarded. By "Supernatant Fraction V" is meant, herein, the fraction identified as such in the Cohn Fractionation Process, or the equivalent fraction produced as a result of the carrying out of an equivalent process on human plasma. The Cohn process, as outlined in Antoniades, id, is reprinted below.

PLASMA

EtOH, 8%
Γ/2 0.14
pH 7.2
Temp. -3°
Protein 5.1%

Supernatant I          Fraction I

EtOH 25%
Γ/2 0.09
pH 6.9
Temp. -5°
Protein 3.0%

Supernatant II + III          Fraction II + III

EtOH 18%
Γ/2 0.09
pH 5.2
Temp. -5°
Protein 1.6%

Supernatant IV-1          Fraction IV-1

EtOH 40%
Γ/2 0.09
pH 5.8
Temp. -5°
Protein 1.6%

Supernatant IV-4          Fraction IV-4

EtOH 40%
Γ/2 0.11
pH 4.8
Temp. -5°
Protein 0.8%

Supernatant V                    Fraction V
Fraction VI (See Fig. 5)         EtOH 10%
                                 Γ/2 0.1
                                 pH 4.5
                                 Temp. -3°
                                 Protein 3%

**Supernatant Fraction V**

Supernatant fluid

EtOH 40%          (impurities, including
Γ/2 0.01           bilirubin - containing
pH 4.8             α-globulin)
Temp. -5°
Protein 2.5%

There have since been, and may be in the future, minor modifications of the process which do not affect the composition of Supernatant Fraction V, e.g., the approved modification of the process adopted September 1955 by the Commonwealth of Massachusetts and described in Appendix 1a (March, 1983) "Procedure for the Fractionation of Human Plasma for the Preparation of Immune Serum Globulin and Normal Serum Albumin (Human)", Commonwealth of Massachusetts Department of Public Health Biological Laboratories. This modified procedure is as follows:

## PLASMA

| | |
|---|---|
| Ethanol | 8% |
| $\Gamma/2$ | 0.14 |
| pH | 7.2 + 0.2 |
| Temp. | −2.5° C $\pm$ 0.25° C |
| Protein | 5.1% |

## SUPERNATANT I                    FRACTION I

| | |
|---|---|
| Ethanol | 25% |
| $\Gamma/2$ | 0.09 |
| pH | 6.85 $\pm$ 0.10 |
| Temp. | −5° C or colder |
| Protein | 3.9% |

Utilized for Research
purposes or discarded

## SUPERNATANT II + III               FRACTION II + III

| | |
|---|---|
| Ethanol | 40% |
| $\Gamma/2$ | 0.09 |
| pH | 6.00 $\pm$ 0.05 |
| Temp. | −5° C |
| Protein | 2.8% |

See Method 9

## FRACTION IV

Utilized for Research
purposes or discarded

## SUPERNATANT IV

### FILTERED

PreFilter Cuno 01A
Cuno 60S
+1/2% (W/V) Filter Aid

| | |
|---|---|
| Ethanol | 40% |
| Γ/2 | 0.11 |
| pH | 4.85 ± 0.05 |
| Temp. | −5.5° C ± 0.5% |
| Protein | 1.6% |

## SUPERNATANT V

(Normally Discarded)

### FRACTION V

| | |
|---|---|
| Ethanol | 10% |
| Γ/2 | 0.01 |
| pH | 4.6 ± 0.1 |
| Temp. | −2.5° C ± 0.5 |
| Protein | 3% |

### FILTERED

Cuno 60S Pads
+ 1/4% (W/V) Filter Aid

### FILTRATE

| | |
|---|---|
| Ethanol | 40% |
| Γ/2 | 0.01 |
| pH | 6.5 ± 0.5 |
| Temp. | −5° C or colder |
| Protein | 2.5% |

## SUPERNATANT

(Discarded)

## REWORKED FRACTION V

(Albumin)

Other features and advantages of the invention will be apparent from the following description of the preferred embodiments thereof, and from the claims.

## Description of the Preferred Embodiments

We now describe preferred embodiments of the invention.

## Media Preparation

The first step in preparing a cell growth medium of the invention is to isolate the protein-rich Supernatant Fraction V from human plasma using Cohn cold ethanol fractionation method 6. In general, this involves a series of successive fractionation steps using aqueous ethanol fractionation solutions; the solutions vary in ethanol concentration, ionic strength, pH, and protein concentration. Each fractionation step, which involves treatment with an appropriate fractionation solution followed by centrifugation, produces a plasma fraction having a distinctive distribution of plasma proteins. The ethanol concentration, ionic strength, pH, and protein concentration of each fractionation solution, as well as the temperature of each fractionation step, are chosen so as to maximize the stability of the fractionated proteins. Fractionation continues until Supernatant Fraction VI is isolated.

Once Supernatant Fraction V has been isolated, it is filtered in one or more ultrafiltration steps to yield a protein fraction in which the proteins hace molecular weights greater than about 30 KD; the proteins consist primarily of albumin (about 80%). The protein fraction is then sterilized to remove bacteria and hepatitis and other viruses by heat treatment at about 60°C for approximately 10-20 hrs, followed by filtration through a sterile filter. The final protein concentration is about 4-10 g protein /100 ml of solution.

The sterilized protein is then mixed with a conventional serum-free medium containing inorganic salts (electrolytes, etc.), amino acids, and vitamins required for cell growth, e.g., RPMI 1640, RPMI plus HEPES, Dulbecco's Modified Medium, etc., to provide the cell growth medium of the invention. The protein concentration in the complete medium is about 100-2000 $\mu$g/ml, preferably about 400$\mu$g/ml.

The resulting medium can be used to grow and maintain a variety of mammaliam cells, including the following general types of cells: 1) lymphoid cells; 2) anchorage-dependent cells; and 3) hybridoma cells. Conventional cell-culturing techniques and conditions are used.

An example of the preparation of a particular cell growth medium follows.

## Fractionation

Human plasma (New York Blood Center) was treated at -3°C with a fractionation solution having the following composition:

a) Ethanol - 8%
b) Ionic Concentration ($\Gamma$/2) - 0.14
c) pH - 7.2.

The protein content of the resulting mixture was 5.1%. The mixture was then separated and the supernatant (Supernatant I) saved.

Supernatant I was treated at -5° C with a fractionation solution having the following composition:

a) Ethanol - 25%
b) Ionic Concentration ($\Gamma$/2) - 0.09
c) pH 6.9.

The protein content of the resulting mixture was 3.0 %. The mixture was then separated and the supernatant (Supernatant II & III) saved.

Supernatant II & III was treated at -5° C with a fractionation solution having the following composition:

a) Ethanol - 18%
b) Ionic Concentration ($\Gamma$/2) - 0.09
c) pH - 6.9.

The protein content of the resulting mixture was 1.6%. The mixture was then separated and the supernatant (Supernatant IV-1) saved.

Supernatant IV-1 was treated at -5° C with a fractionation solution having the following composition:

a) Ethanol - 40%
b) Ionic Concentration ($\Gamma$/2)-0.09
c) pH - 5.8.

The protein content of the resulting solution was 1.0%. The mixture was then spearated and the supernatant (Supernatant IV-4) saved.

Supernatant IV-4 was treated at -5° C with a fractionation solution having the following composition:

a) Ethanol - 40%

b) Ionic Concentration (Γ/2) - 0.11

c) pH - 4.8.

The protein content of the resulting solution was 0.8%. The mixture was then separated, the supernatant discarded, and the protein-rich fraction (Fraction V) saved.

Fraction V, containing mostly albumin, was treated at -3° C with a fractionation solution having the following composition:

a) Ethanol - 10%

b) Ionic Concentration (Γ/2) - 0.1

c) pH - 4.5.

The protein content of the mixture was 3%. The mixture was then separated and the supernatant retained; this was Supernatant Fraction V.

Filtration

Supernatant Fraction V obtained from the above-described fractionation procedure was filtered using an ultrafiltration membrane with a 30 KD cut-off; all material under 30 KD was discarded. The material above 30 KD was then heated for 10-20 hrs. at 60° C to kill hepatitis and other viruses. The material remained stable during this heat treatment, with no gellation of proteins. Following heat treatment, the material was filtered through a sterile nylon 66 0.1 μm membrane filter to remove bacteria. The filtration yielded a protein fraction having a protein concentration of 4 g protein /100 ml of solution.

Cell Growth Medium Preparation

10 ml of the above-described protein fraction containing glutamine and appropriate antibodies was added to 990 ml of RPMI 1640 serum-free media (Biofluids, Inc. - Rockville, Md. catalog #102) to form a cell-growth medium. The concentration of protein in the medium was 400 μg protein/ml medium. The medium was used to grow and maintain the following cell lines using conventional techniques:

A. Lymphoids

1. HL-60 (ATCC CCL240) - human promyelocytic leukemia
2. CCRF-CEM (ATCC CCL119) human acute lymphobcostic leukemia
3. U-937 (ATCC CRL1593), human histiocytic lymphoma
4. K562 (ATCC CCL243), human chronic myelogenous leukemia
5. MO-B (ATCC CCL245), human hairy-cell leukemia variant
6. EBK, human EB transformed B-cell
7. NAMACUA, human EB transformed B-cell

B. Hybridomas

1. 2B4, mouse T-cell
2. TH2.2, (M12.4.1) B-cell lymphoma x B6 mouse spleen
3. TH2.5, M12.4.1 x B6 mouse Aplic
4. A₂0, B cell lymphoma mouse
5. AR40BC4AI mouse
6. AR32CAJIC3 mouse
7. TNP14Z mouse

C. Anchorage - Dependent

1. VERO, African green monkey kidney
2. glioblastama, A172
3. fibrosorasoma, T1080
4. ostrosoream, U20S
5. WI-38-normal human lung
6. GM2767115-normal human lung
7. HFS-1-normal human foreskin (fibroblasts)

Other embodiments are within the following claims.

**Claims**

1. A cell culture medium comprising Cohn Supernatant Fraction V.
2. The cell culture medium of claim 1, said medium being virus-free.
3. A method of culturing mammalian cells comprising contacting said cells with the medium of claim 1.